# EUROPEAN PATENT SPECIFICATION

(11) **EP 1 311 853 B1**
(45) Date of publication and mention of the grant of the patent: **10.10.2007**
(21) Application number: 01950862.1
(22) Date of filing: 03.07.2001
(51) Int. Cl.: G01N 33/543, G01N 33/552, G01N 33/545, G01N 33/547

(54) **COMBINATION OF MICROPOROUS MEMBRANE AND SOLID SUPPORT FOR MICRODIAGNOSTICS**
ZUSAMMENSETZUNG VON MIKROPORÖSER MEMBRAN UND FESTPHASE FÜR MIKRODIAGNOSTIKA
COMBINAISON DE MEMBRANE MICRO-POREUSE ET DE SUPPORT SOLIDE DESTINEE AU MICRO-DIAGNOSTIC

(30) Priority: 06.07.2000 US 216390 P
(43) Date of publication of application: 21.05.2003
(73) Proprietor: 3M Innovative Properties Company, St. Paul, Minnesota 55133-3427 (US)
(72) Inventor: MEYERING, Mark, Middlefield, CT 06455 (US); AMIN, Murtaza, UT 84109 (US); OSTREICHER, Eugene, Farmington, CT 06032 (US)
(74) Representative: Charig, Raymond Julian
(86) International application number: PCT/US2001/021210
(87) International publication number: WO 2002/004477

(56) References cited:
- US-A- 3 874 986
- US-A- 3 876 738
- US-A- 5 006 287
- US-A- 5 124 128
- US-A- 5 420 047

## Description

### Background of the Invention

The present invention relates to a multi-cell substrate useful for carrying a microarray of biological polymers on the surface thereof and, more particularly to a multi-cell substrate having a porous membrane formed by a phase inversion process effectively attached by covalent bonding through a surface treatment to a substrate that prepares the substrate to sufficiently, covalently bond to the microporous membrane formed by a phase inversion process such that the combination produced thereby is useful in microarray applications and, most particularly, to a multi-cell substrate wherein a porous nylon multi-cell substrate is covalently bonded to a solid base member, such as, for example, a glass or Mylar microscope slide, such that the combination produced thereby is useful in microarray applications and to a process for producing such multi-cell substrates.

A variety of methods are currently available for making arrays of biological macromolecules, such as, for example, arrays of nucleic acid molecules or proteins. One method for making ordered arrays of DNA on a porous membrane is a "dot blot" approach. In this method, a vacuum manifold transfers a plurality, e.g., 96, aqueous samples of DNA from 3 millimeter diameter wells to a porous membrane. A common variant of this procedure is a "slot-blot" method in which the wells have highly-elongated oval shapes.

The DNA is immobilized on the porous membrane by baking the membrane or exposing it to UV radiation. This is a manual procedure practical for making one array at a time and usually limited to 96 samples per array. "Dot-blot" procedures are therefore inadequate for applications in which many thousand samples must be determined.

A more efficient technique employed for making ordered arrays of genomic fragments (e.g., PCR products) uses an array of pins dipped into the wells, e.g., the 96 wells of a microtitre plate, for transferring an array of samples to a substrate, such as a porous membrane. One array includes pins that are designed to spot a membrane in a staggered fashion, for creating an array of 9216 spots in a 22 x 22 cm area (Lehrach, et al., 1990). A limitation with this approach is that the volume of DNA spotted in each pixel of each array is highly variable. In addition, the number of arrays that can be made with each dipping is usually quite small.

An alternate method of creating ordered arrays of nucleic acid sequences is described by Pirrung, et al. (1992), and by Fodor, et al. (1991). The method involves synthesizing different nucleic acid sequences at different discrete regions of a support. This method employs elaborate synthetic schemes, and is generally limited to relatively short nucleic acid sample, e.g., less than 20 bases. A related method has been described by Southern, et al. (1992).

Khrapko, et al. (1991) describes a method of making an oligonucleotide matrix by spotting DNA onto a thin layer of polyacrylamide. The spotting is done manually with a micropipette.

Roda, et al. (2000) describe a method for producing bidimensional arrays of horseradish peroxidase (HRP) on cellulose paper using a commercial ink-jet printer at a density of 10-100 spots/cm².

None of the methods or devices described in the above prior art are designed for mass fabrication of microarrays characterized by (i) a large number of micro-sized assay regions separated by a distance of 50-200 microns or less, and (ii) a well-defined amount, typically in the picomole range, of analyte associated with each region of the array.

Furthermore, current technology is directed at performing such assays one at a time to a single array of DNA molecules. For example, the most common method for performing DNA hybridizations to arrays spotted onto porous membrane involves sealing the membrane in a plastic bag (Maniatis, et al., 1989) or a rotating glass cylinder (Robbins Scientific) with the labeled hybridization probe inside the sealed chamber. For arrays made on non-porous surfaces, such as a microscope slide, each array is incubated with the labeled hybridization probe sealed under a coverslip. These techniques require a separate sealed chamber for each array, which makes the screening and handling of many such arrays inconvenient and time intensive.

Abouzied, et al. (1994) describes a method of printing horizontal lines of antibodies on a nitrocellulose membrane and separating regions of the membrane with vertical stripes of a hydrophobic material. Each vertical stripe is then reacted with a different antigen and the reaction between the immobilized antibody and an antigen is detected using a standard ELISA colorimetric technique. Abouzied's technique makes it possible to screen many one-dimensional arrays simultaneously on a single sheet of nitrocellulose. Abouzied makes the nitrocellulose somewhat hydrophobic using a line drawn with PAP Pen (Research Products International). However, Abouzied does not describe a technology that is capable of completely sealing the pores of the nitrocellulose. The pores of the nitrocellulose are still physically open and so the assay reagents can leak through the hydrophobic barrier during extended high temperature incubations or in the presence of detergents, which makes the Abouzied technique unacceptable for DNA hybridization assays.

Porous membranes with printed patterns of hydrophilic/hydrophobic regions exist for applications such as ordered arrays of bacteria colonies. QA Life Sciences (San Diego, Calif.) makes such a membrane with a grid pattern printed on it. However, this membrane has the same disadvantage as the Abouzied technique since reagents can still flow between the gridded arrays making them unusable for separate DNA hybridization assays.

Pall Corporation makes a 96-well plate with a porous filter heat sealed to the bottom of the plate. These plates are capable of containing different reagents in each well without cross-contamination. Each well is intended to hold only one target element. Furthermore, the 96 well plates are at least 1 cm thick and prevent the use of the device for many calorimetric, fluorescent and radioactive detection formats which require that the membrane lie flat against the detection surface.

Hyseq Corporation has described a method of making an "array of arrays" on a non-porous solid support for use with their sequencing by hybridization technique. The method described by Hyseq involves modifying the chemistry of the solid support material to form a hydrophobic grid pattern where each subdivided region contains a microarray of biomolecules. Hyseq's flat hydrophobic pattern does not make use of physical blocking as an additional means of preventing cross-contamination.

Several patents have described the use of multi-cell substrates in microarray applications. These include U.S. Patent No. 5,919,626 entitled, "Attachment of unmodified nucleic acids to silanized solid phase surfaces"; U.S. Patent No. 5,667,976 entitled, "Solid supports for nucleic acid hybridization assays" and U.S. Patent No. 5,760,130 entitled "Aminosilane/carbodiimide coupling of DNA to glass substrate".

Multi-cell substrates are well known in the art. Schleicher & Schuell have attempted to attach nylon membrane to a glass slide using glue or similar adhesive in their commercially available CAST^{™} slides. However, the layer of glue or adhesive adds additional thickness to the nylon membrane/glass slide combination, and the gluing/adhesive process may require the use of a scrim-reinforced nylon membrane. The extra thickness of the overall nylon membrane/glass slide combination caused by the glue/adhesive and the reinforcing scrim is a disadvantage in microarray applications. Additionally, the scrim makes the surface of the membrane of the nylon membrane/glass slide combination uneven and less than ideal from a cosmetic standpoint. Even further, the chemistry of the glue or adhesive used to attach the nylon membrane to the glass slide is not necessary optimal to effectuate the combination, nor is it necessarily compatible with the biomolecules or analytes for which the product is intended to receive, as it may interfere or react with the analyte.

Similarly, other products known to be currently commercially available include: Modified glass that binds nucleic acids or proteins without the use of a membrane; Corning GAPS Slides, such as, for example CMT-GAPS^{™} coated slides; Nitrocellulose porous membrane cast onto glass, available from Schleicher & Schuell as FAST^{™} Slides; Scrim-reinforced nylon glued or adhered to a glass substrate and Schleicher & Schuell CAST^{™} Slides. Detailed descriptions of these commercially available products are readily available from the respective manufacturers and are known in the art.

However, in microarray applications, binding nucleic acids or proteins directly to a glass substrate has certain disadvantages. Specifically, a considerably smaller surface area for binding the nucleic acids or proteins is available than with a comparably sized microporous membrane/glass slide combination. The larger the binding surface area, the better the signal strength of the biomolecules or analytes, thereby allowing for the detection of smaller samples of biomolecules or analytes. Also, the porous membrane portion of the microporous membrane/glass slide combination naturally adsorbs the biomolecules or analytes and holds them in place on the microporous membrane/glass slide combination, whereas without the microporous membrane portion of the slide, the biomolecules or analytes would just sit on top of a glass surface, as there is no adsorption of the biomolecules or analytes. It is also likely that the efficiency of immobilization of biomolecule on the glass is substantially less than 100 %, and may be less than 50 %, when compared to immobilization of the target on nylon. This is important, in that the subsequent detection steps require as much of the possible analyte, or target biomolecule, to be available for (in a DNA detection example) hybridization with the labeled probe. Following the immobilization, there are typically several liquid immersion steps including blocking, washing, hybridization buffer exposure, etc. Each step has the potential of removing analyte from the glass surface, and decreasing the potential strength of the signal. Nylon is generally regarded as having the highest biomolecule binding efficiency when compared to other the commercially available polymer or other -treated substrates. Nylon is also regarded as providing highest accessibility of the functional groups of the analyte thus bound to the nylon surfaces.

Nylon membranes, a specific species of microporous membrane, formed by a phase inversion process, have some advantages over nitrocellulose membranes in that nylon is naturally hydrophilic. Nylon membranes also have a greater protein and DNA binding capacity than nitrocellulose. This increased binding capacity means better signal strength and lower detection thresholds in assays.

Nylon membrane pore structure is more easily controllable than nitrocellulose membrane pore structure, and is more physically robust than the nitrocellulose membranes. Nitrocellulose is more brittle than the nylon membrane, has more pore variability and is extremely flammable. The physical weakness, variability and flammability of the nitrocellulose membranes combine to make nitrocellulose membrane more expensive to manufacture than nylon membrane.

As discussed above, there are at least three main disadvantages to scrim-reinforced nylon glued or otherwise adhered to a glass substrate. First, the glue or adhesive layer adds additional thickness to the combination scrim-reinforced nylon/glass slide. The arraying robots that blot the nylon membranes have narrow spatial tolerances, and any additional thickness represents additional uncertainty about accurate positioning of the combination scrim-reinforced nylon/glass slide relative to the arraying robots. The second, and more important, disadvantage is that the scrim-reinforced membrane on the combination scrim-reinforced nylon/glass slide has an irregular surface on the micro scale. This is an important cosmetic problem since the spot sizes made on the membrane are on a similar scale. Finally, the glue/adhesive and the analyte may not be compatible. Specifically, the adhesive which contains an excess of functionalized moieties for attachment can indiscriminately bind the analyte in a way which makes it unavailable for detection; either by binding to the molecule preventing (in the DNA example) hybridization, or by reversibly binding to the analyte such that the attachment is not permanent, and the analyte is sloughed off in the liquid immersion steps prior to detection. Finally, the adhesive itself can be degraded in the multistep processes leading to detection, and become, by extraction or other means, a mobile species. The adhesive fragment, if bound to the analyte, may be displaced to a location or area beyond the location of detection, or itself become part of a false background signal, depending on the type of detection being performed.

In these types of multi-cell substrates or slides, it is useful to have a nylon microporous layer that is flat, uniform, and is as thin as possible. In the case of charge modified slides, the degree of charge modification must be uniform over the entire slide surface. In the environment of use, as envisioned for the innovative slides described in the present application, the bond between the nylon and the base member, such as, for example, a glass slide or Mylar sheet, must stand up to water, NaOH, sodium dodecyl sulfate, and other harsh chemicals for prolonged periods of time and at high temperatures. Because of the high air pressure generated between the nylon membrane layer and the glass substrate when the nylon membrane is wetted, the bond therebetween must also be physically strong.

Thus, there is a need for a relatively thin, multi-cell substrate useful for Micro-Analytical Diagnostic Applications. Such a multi-cell substrate structure should be naturally hydrophilic. Such a multi-cell substrate's properties should be easily controlled. Such a multi-cell substrate should be more physically robust than the nitrocellulose membrane slides of the prior art. Such a multi-cell substrate should be relatively easily manufactured. Such a multi-cell substrate should at least minimize, if not eliminate, any glue/adhesive layer between the membrane and the solid substrate which adds thickness to the membrane/substrate combination. Such a multi-cell substrate should have a porous membrane formed by a phase inversion process surface treatment for a substrate that prepares the substrate to operatively, covalently bond to a microporous membrane formed by a phase inversion process such that the combination produced thereby is useful in microarray applications. Such a multi-cell substrate should include a surface treatment that has no discernable finite thickness or mass which could add nonuniformity to the overall thickness of the multi-cell substrate having a porous membrane formed by a phase inversion process useful in microarray applications. Such a multi-cell substrate should include a surface treatment that at least minimizes, if not eliminates, the participation of this treatment in the binding or detection of nucleic acid or protein analytes by a multi-cell substrate having a porous membrane formed by a phase inversion process useful in microarray applications. Such a multi-cell substrate should include a porous membrane formed by a phase inversion process useful in microarray applications which includes a surface treatment to the solid substrate that minimizes the interference of the substances used to connect the solid substrate portion to the porous membrane portion used for the detection of analytes. Such a multi-cell substrate should include a porous membrane formed by a phase inversion process useful in microarray applications which includes a surface treatment that eliminates nonuniformity of the overall thickness of the substrate/membrane combination structure which is associated with using a third component having a finite thickness or mass as the connecting agent. Such a multi-cell substrate should have a regular surface on the micro scale. Such a multi-cell substrate should eliminate compatibility issues between the glue/adhesive and the analyte. Such a multi-cell substrate should be economically produced. US 5 420,047 discloses a phase-inversion membrane for immunoassays,

### SUMMARY OF THE INVENTION

An object of the present invention is to provide a multi-cell substrate having a porous membrane formed by a phase inversion process surface treatment for a substrate that prepares the substrate to operatively, covalently bond to a microporous membrane formed by a phase inversion process such that the combination produced thereby is useful in microarray applications.

Another object of the present invention is to provide a surface treatment that has no discernable finite thickness or mass which could add nonuniformity to the overall thickness of a multi-cell substrate having a porous membrane formed by a phase inversion process useful in microarray applications.

A further object of the present invention is to provide a surface treatment that minimizes participation in the binding or detection of nucleic acid or protein analytes of a multi-cell substrate having a porous membrane formed by a phase inversion process useful in microarray applications.

Yet a further object of the present invention is to provide a multi-cell substrate having a porous membrane formed by a phase inversion process useful in microarray applications which includes a surface treatment that minimizes the interference of the substances used to connect the solid substrate portion to the porous membrane portion thereof with the detection of analytes.

Yet another object of the present invention is to provide a method for fabricating a multi-cell substrate having a porous membrane formed by a phase inversion process surface treatment for a substrate that prepares the substrate to sufficiently, covalently bond to a microporous membrane formed by a phase inversion process such that the combination produced thereby is useful in microarray applications.

Still another object of the present invention is to provide a multi-cell substrate having a porous membrane formed by a phase inversion process useful in microarray applications which includes a surface treatment that eliminates nonuniformity of the overall thickness of the substrate/membrane combination structure which is associated with using a third component having a finite thickness or mass as the connecting agent.

In accordance with these and further objects, one aspect of the present invention includes a multi-cell substrate, useful for carrying a microarray of biological polymers comprising: a microporous membrane formed by a phase inversion process; a non-porous substrate; and a surface treatment, operatively positioned between the microporous membrane and the non-porous substrate, for sufficiently covalently bonding the non-porous substrate to the microporous membrane wherein the combination multi-cell substrate produced thereby is useful in microarray applications.

Another aspect of the present invention includes a method of fabricating a multi-cell substrate useful for carrying a microarray of biological polymers comprising the acts of: providing a non-porous substrate; providing a microporous membrane formed by a phase inversion process; providing a surface treatment; applying the surface treatment to the non-porous substrate; and intermingling the non-porous substrate having the surface treatment with the microporous membrane such that the non-porous substrate is sufficiently covalently bonded to the microporous membrane wherein the combination produced thereby is useful in microarray applications.

Another aspect of the present invention may include a posttreatment of the microporous membrane such that the membrane contains a greater positive charge; such a treatment is useful in augmenting the microporous membrane's ability to retain biological polymers, which predominantly are negatively charged.

### BRIEF DESCRIPTION OF THE DRAWINGS

Figure 1 is a graphic depiction of representative organosilanes useful with the present application;
Figure 2 is a representative graphic depiction of the hydrolysis of an organosilane to produce an organosilanol useful with the present application;
Figure 3 is a representative graphic depiction of a silanol condensation reaction in which the silanol in solution condenses with the silanol on the glass surface, expelling water and creating the treated glass surface of the present application;
Figure 4A is a representative graphic depiction of a reaction of an epoxy with an amino functional group useful with the present application;
Figure 4B is a representative graphic depiction of a reaction of an epoxy with a carboxyl functional group useful with the present application;
Figure 5A is a representative graphic depiction of a bond between nylon and glass resulting from using 3-aminopropyl triethoxysilane and polyamido polyamine epichlorohydrin polymer useful with the present application;
Figure 5B is a representative graphic depiction of a bond between nylon and glass resulting from using 1-carbomethoxy-decyl-dimethyl chlorosilane and polyamido polyamine epichlorohydrin polymer useful with the present application;
Figure 5C is a representative graphic depiction of a bond between nylon and glass resulting from using glycidoxypropyltrimethoxysilane useful with the present application;
Figure 6 is a graphic depiction of a representative metal hemi-drum depicting the placement of glass slides thereon useful with the present application; and
Figure 7 is a graphic depiction of a representative metal hemi-drum depicting the placement of a sheet of Mylar thereon useful with the present application.

### DETAILED DESCRIPTION OF REPRESENTATIVE EMBODIMENTS

Unless indicated otherwise, the terms defined below have the following meanings:

"Analyte" or "analyte molecule" refers to a molecule, typically a macromolecule, such as a polynucleotide or polypeptide, whose presence, amount, and/or identity are to be determined. The analyte is one member of a ligand/anti-ligand pair.

"Analyte-specific assay reagent" refers to a molecule effective to bind specifically to an analyte molecule. The reagent is the opposite member of a ligand/anti-ligand binding pair.

An "array of regions on a solid support" is a linear or two-dimensional array of preferably discrete regions, each having a finite area, formed on the surface of a solid support.

A "microarray" is an array of regions having a density of discrete regions of at least about 100/cm², and preferably at least about 1000/cm². The regions in a microarray have typical dimensions, e.g., diameters, in the range of between about 10-250 µm, and are separated from other regions in the array by about the same distance.

A "phase inversion process" is meant to encompass the known art of porous membrane production techniques which involve phase inversion in its various forms, to produce "phase inversion membranes." By "phase inversion membranes" it is meant a porous membrane that is formed by the gelation or precipitation of a polymer membrane structure from a "phase inversion dope." A "phase inversion dope" consists of a continuous phase of dissolved polymer in a good solvent, co-existing with a discrete phase of one or more non-solvent(s) dispersed within the continuous phase. In accordance to generally acknowledged industry practice, the formation of the polymer membrane structure generally includes the steps of casting and quenching a thin layer of the dope under controlled conditions to effect precipitation of the polymer and transition of discrete (non-solvent phase) into a continuous interconnected pore structure. In one manner of explanation, this transition from discrete phase of non-solvent (sometimes referred to as a "pore former") into a continuum of interconnected pores is generally known as "phase inversion." Such membranes are well known in the art. Occasionally, such membranes and processes will be called "ternary phase inversion" membranes and processes, with specific reference to the ability to describe the composition of the dope in terms of the three major components; polymer, solvent, and non-solvent(s). The presence of the three major components comprise the "ternary" system. Variations of this system include: liquid phase inversion, evaporative phase inversion, thermal phase inversion (where dissolution is achieved and sustained at elevated temperature prior to casting and quenching), and others.

Composite microarray slides comprise a porous nylon or other polymer membrane bound to a solid backing, typically a glass microscope slide. Microarray slides are used in gene sequencing and expression analysis applications where thousands of hybridization assays are performed on the surface of a single microarray slide.

When a microporous nylon membrane formed by a phase inversion process is still wet from casting, the nylon membrane has a greater thickness than after being dried. If the membrane is stretched out over a surface and then dried, the nylon membrane shrinks in the direction of thickness. The nylon membrane also binds tightly to the surface it contacts. If the nylon membrane has been dried once and then rewetted, the nylon membrane does not exhibit the binding property described above. More importantly, the nylon membrane loses the binding property once the nylon membrane gets wet after having been tightly bound to a surface.

Given the above characteristic of nylon membrane, it was decided to attempt to find mechanisms to attach the nylon membrane to a substrate, such as, for example, glass, such that the bond between the nylon membrane and the substrate would remain intact after being exposed to various known severe conditions experienced in actual practice. For example, the nylon/solid composite slide should withstand immersion in an 80°C, 1% sodium dodecyl sulfate (SDS) solution.

For the purposes of the present application, an organosilane has the formula:

RSi(X)_{3-N}A_{N},

where X is an ethoxy, methoxy, or chloride group, and R is a functional group that interacts with nylon, or with an intermediate substance capable of bonding to nylon. The 'A' group is an additional unreactive group that may or may not be present (depending on whether N is 0, 1, or 2). In the case of nylon, as examples, R could contain ureido, amino, carboxy, epoxy or other functional groups capable of bonding directly to nylon or to some intermediate substance that is capable of bonding to nylon.

Based on various experiments, some organosilane treatments appeared to be very feasible to bind porous nylon membrane to glass in a way that does not affect the chemical or physical properties of the membrane.

An effective method for bonding the nylon to the solid substrate has been developed. In this method, the glass slides were first immersed in a 2% solution of 3-aminopropyl triethoxysilane (an aminosilane) in aqueous ethanol for two minutes. Following immersion, the slides were then briefly rinsed with ethanol to remove particulates, then placed into an oven for about ten (10) minutes at about 120° C. After the treated slides were cured for about a day at room temperature, the slides were immersed in an about 3.5% solids solution of a polyamido polyamine epichlorohydrin resin (specifically 'Resicart E', manufactured by Ciba-Geigy) containing tetraethylene pentamine (TEPA) for about one hour. The slides were then rinsed with DI water, laminated with the membrane, and further cured in an oven at about 120°C for about one hour. The newly prepared composites were then cured at room temperature for at least a day before being tested. This method was designated Aminosilane-Resicart-Coat (ARC).

Though Resicart E is inherently positively charged, the functional surfaces of the composites do not show properties of the positive charge of Resicart E. This is because the Resicart E is only present at the interface between the nylon and the glass, and not present as a coating on the internal or external surfaces of the nylon membrane, that is, Resicart E is not functioning as a surface charge modifier to nylon.

To charge modify the ARC composites, a separate charge modification step is required. For example, the cured samples were immersed in about a 3.5% solids Resicart E with TEPA for about 3 minutes, rinsed well with DI water, shaken to remove excess water (eliminating the gloss of water from the surface), and heated in an oven at about 60°C until dry. Other methods of charge-modifying the nylon portion of the membrane are possible, for example, a spray, brush, or foam application of charge modifier on the upper surface of the membrane. Alternatively, a pre-modified layer of nylon microporous membrane can be produced by direct addition of charge modifying chemistry to the nylon dope.

Incubation of membrane-glass composite slide in a 1.0% SDS solution at about 80°C, did not separate the uncharged ARC samples from the substrate. The cosmetic appearance and bond strength of the above mentioned unmodified slides after immersion were generally good. The bond strength was stronger than the membrane's tensile strength even after the composite was subjected to near-boiling 1% SDS for about one hour.

The aforementioned charge modified ARC-samples also stayed bonded, but the bond strength tended to be weaker than the unmodified slides. The longer the charge modified ARC composites cured (at room temperature) before being tested, the stronger the bond. Results indicate that if they were tested a day after being charge treated, they had only fair bond strength after SDS exposure. If allowed to cure for a week or more, the composites' bond strength tended to be very good. When submerged in dilute solution of metanil yellow dye (a negatively charged compound), the charged ARC samples showed uniform binding of the dye on the surface, indicating even positive charge distribution. The interface layer of the composites (charged and uncharged) showed a high binding of dye, too, indicating that Resicart E is present at the interface (as expected). Therefore, all surfaces (internal and external) of the full thickness of the nylon structure have been charge modified. It should be possible to restrict the charge modification to the upper surface by a different application technique, as mentioned above.

While not wishing to be bound by theory, it is presently believed that the following describes the chemistry controlling the bonding of the nylon to the glass in the nylon/glass composite slides described above and in the Examples.

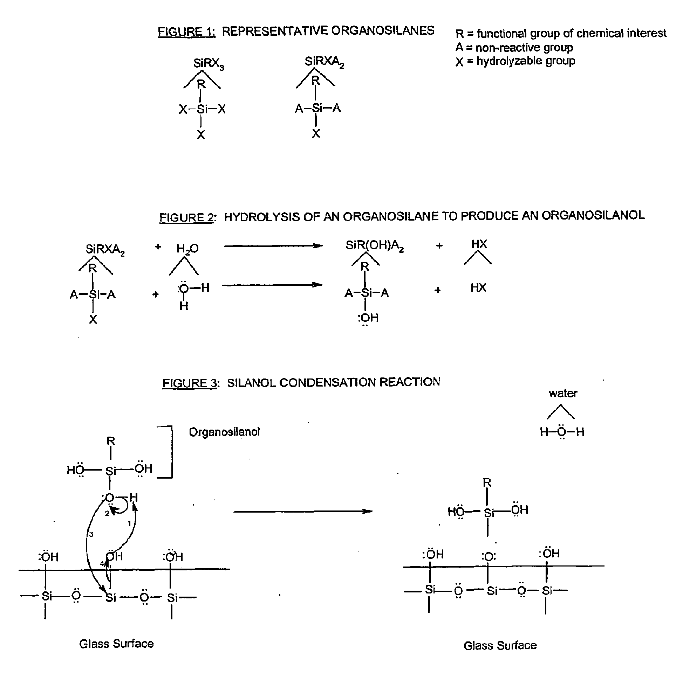

As illustrated in Figure 1, in the first step of bonding the nylon to the glass substrate, about 2 mL of an organosilane is mixed into a solution containing about 95 mL ethanol and about 5 mL water. As shown, the representative organosilane contains four functional groups.

Concerning the present application, the chemistry of the single 'R' functional group is of particular interest. Of the remaining three functional groups on the organosilane, at least one is a hydrolyzable 'X' group. In the present application, the representative organosilane may or may not contain functional groups of other types than the 'R' functional group (which will be defined) and the 'X' functional group (which is a ethoxy-, a methoxy-, or a chloride, any of which is sufficient for the purposes of the present application). If the organosilane does contain other kinds of functionalities (most often a hydrogen or an alkyl group), they are nonreactive and are represented by an 'A' in the drawings.

As illustrated by the reaction depicted in Figure 2, the water in the solution with the organosilane hydrolyzes the X functional groups and produces an organosilanol. This reactive process takes at most about five minutes.

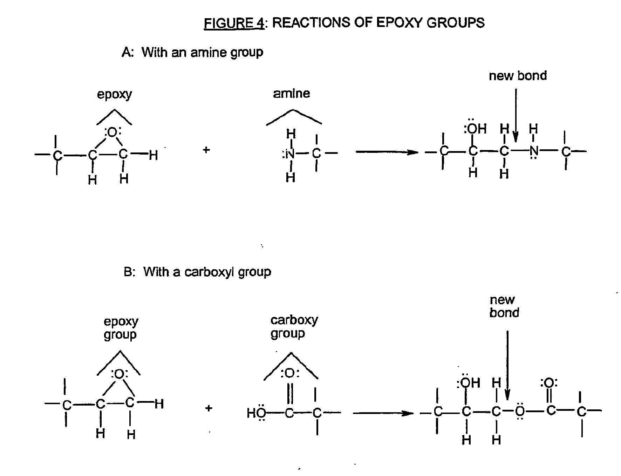

As shown in Figure 3, once the organosilanol is formed, the solution reacts with glass. As illustrated, the organosilanol bonds to the glass surface, giving the glass the surface chemistry of the 'R' functional group.

If the 'R' is an amino or a carboxyl functional group, the glass slide is then exposed to about a 3.5% solids solution of a polyamido polyamine epichlorohydrin resin. In this reaction, an epoxy group on the resin polymer bonds with an amino functional group or a carboxyl functional group according to the illustrations in Figures 4A and 4B, respectively.

The other end of the polyamido polyamine epichlorohydrin polymer has another epoxy functional group capable of bonding to amino or carboxyl functional groups present in nylon.

At this point in the process, the wet-as-cast nylon membrane is placed on top of the wetted, treated glass slides, stretched and clipped into place. After drying for about one hour at about 120°C, the membrane dries, thereby bonding to the glass surface and the epoxy functional groups of the epichlorohydrin polymer bond to amino or carboxyl functional groups on the nylon.

This reaction proceeds as illustrated in Figure 4A and Figure 4B, according to whether the group is an amino functional group or a carboxyl functional group, respectively.

If the 'R' functional group of the organosilanol initially contains an epoxy functional group, the 'R' functional group of the organosilanol can bond directly with the nylon without exposure to polyamido polyamine epichlorohydrin polymer. As before, the epoxy group bonds to either amino functional groups or to carboxyl functional groups on the nylon, as illustrated in Figures 4A and 4B. The nylon membrane is stretched over the membrane and clipped and dried as described above.

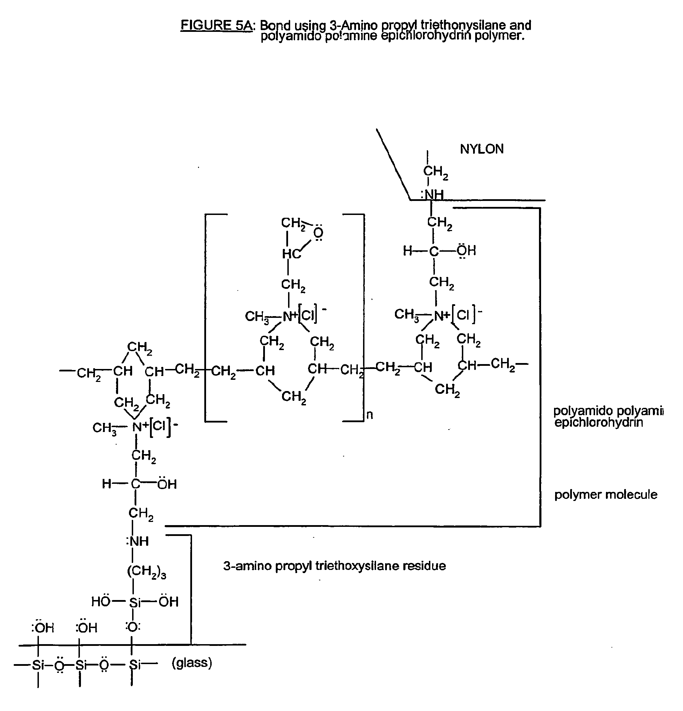

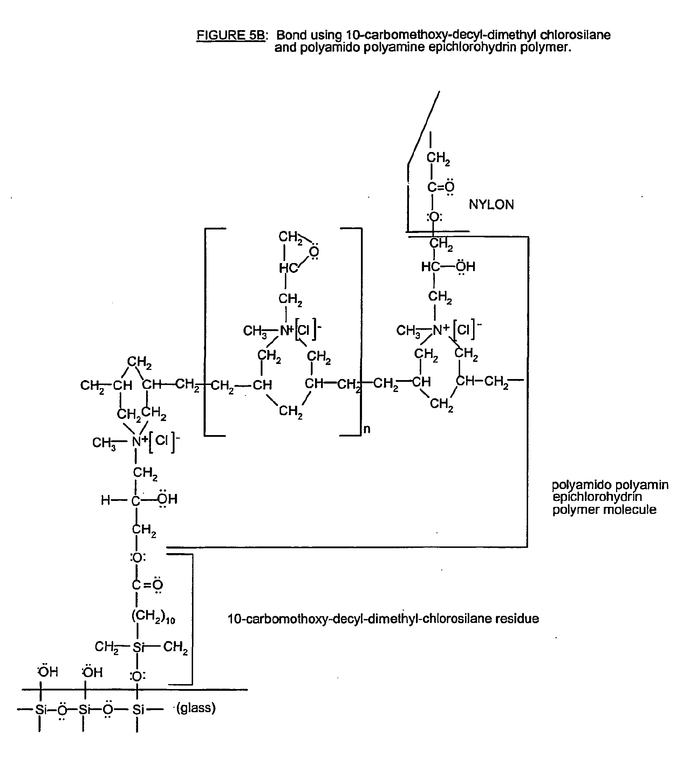

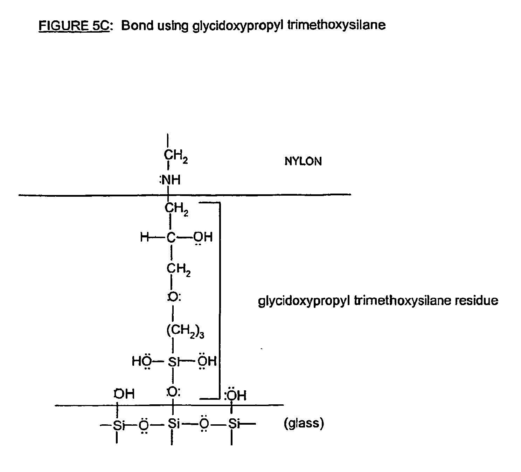

Figure 5 illustrates the final chemical structure of the nylon/glass composite slide depending on the particular kind of functional group the 'R' group represents. Figure 5A illustrates a nylon/glass composite slide in which the 'R' group ends in an amino functional group (specifically, the silane is 3-aminopropyl triethoxysilane). Figure 5B illustrates a nylon/glass composite slide in which the 'R' group ends in a carboxyl functional group (specifically 10-carbomethoxy-decyl-dimethyl chlorosilane). In Figures 5A and 5B, note that the polyamido polyamine epichlorohydrin polymer molecule forms a bridge between the organosilane end group and the nylon.

Figure 5C illustrates a composite in which the 'R' group is an epoxy functional group (specifically glycidoxypropyl trimethoxysilane). Notice that there is no polymer molecule bridging between the organosilane end group and the nylon group.

From an analysis of the nylon/glass composite slides made in accordance with the following examples, a significant portion of the glass and the nylon are in direct contact thereby avoiding a complete separate layer of material between the two slide components. In this manner, the bonding of nylon to glass has been accomplished without the use of an adhesive or gluing layer having any appreciable thickness.

The general procedure for producing multi-cell substrate useful for carrying a microarray of biological polymers on the surface thereof and specifically a nylon multi-cell substrate operatively connected to a glass slide is described below.

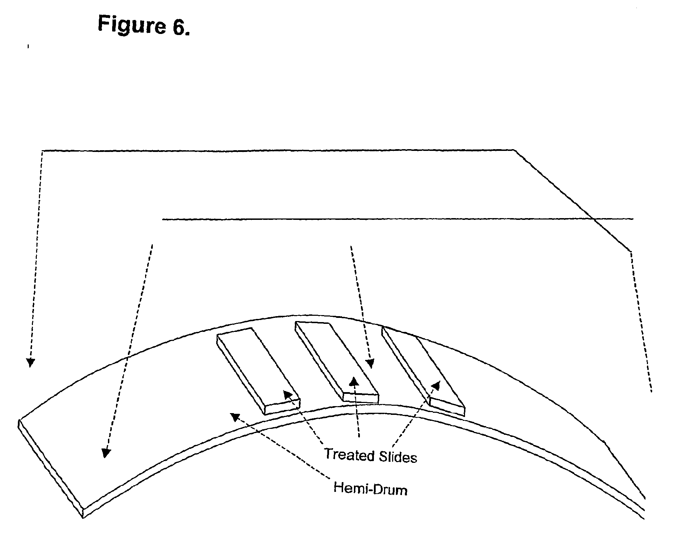

A metal hemi-drum, useful in the production of such slides, is illustrated in Figure 6. It is advantageous to use a metal drum having an outside surface which has been pre-coated with a permanent Teflon coating (such as in non-stick skillets). Using the metal hemi-drum of Figure 6, the slides treated as described above are placed thereon. Next, the surface that will interface with the wet-as-cast-nylon membrane of each treated slide is covered with DI water. An amount of the wet-as-cast-nylon membrane sufficient to cover each treated slide is stretched and positioned over the treated slides, making sure that there are no air bubbles between the glass and the wet-as-cast-nylon membrane. Once the wet-as-cast-nylon membrane is positioned over the treated slides, the wet-as-cast-nylon membrane is secured in position using conventional devices, such as, for example, clips.

The wet-as-cast-nylon membrane/glass slide combinations were placed in a convection oven for a period of about one hour at about one hundred twenty (120°) C. Upon completion of the drying process, the glass to membrane bond has been formed, and the attachment is strong. The combined sheet of membrane plus glass slides is easily peeled off of the teflon coated metal drum, and then the excess nylon membrane is trimmed from the glass slides, with any suitable trimming technique (razor cut, die cut, shear cut, etc.) as is known in the art.

Thus, the following examples illustrate, but in no way are intended to limit, the present invention.

### Example 1

Production of Nylon/Glass Composite slides useful as a multi-cell substrate for carrying a microarray of biological polymers was carried out as follows:

This example describes the process for producing a sample batch of the nylon/glass composite slides. The nylon/glass composite slides which were produced were comprised of a thin (~4 mil) layer of porous nylon membrane operatively bound to the surface of a three-inch (3") by one-inch (1") glass microscope slide. Such slides have proven operable as a multi-cell substrate useful for carrying a microarray of biological polymers.

The process was initiated by preparing an about 100mL solution of about 95% ethanol and about 5% water (percent by volume). About 2 mL of 3-aminopropyl triethoxysilane (made by United Chemicals, Cat .# A0750) was added to the above solution. The combined solution was mixed thoroughly, and was allowed to sit for about five minutes.

Four VWR Brand MicroSlides (part # 48300-025) were placed in an evaporating dish and the 3-aminopropyl triethoxysilane solution was poured into the evaporating dish with the four VWR Brand MicroSlides. The four VWR Brand MicroSlides remained submerged in the 3-aminopropyl triethoxysilane solution for about two minutes. To reduce the possibility of contamination, the four VWR Brand MicroSlides were always handled by personnel wearing gloves.

The 3-aminopropyl triethoxysilane solution was drained from the evaporating dish, and the four VWR Brand MicroSlide were briefly rinsed for about 5 seconds each with ethanol. The four VWR Brand MicroSlides were then wiped dry with a paper towel. During the drying procedure, care was taken not to scratch the surface of the four VWR Brand MicroSlides. It was found during this step that excessive rinsing of microslides with ethanol disrupts the temporary hydrogen bonding of the silane prior to cure, resulting in diminished bonding. At this time, the four VWR Brand MicroSlides were inspected for visual blemishes or other imperfections. Any of the four VWR Brand MicroSlides with visual blemishes or other imperfections were rejected and not used.

If all slides passed the visual inspection, the four VWR Brand MicroSlides were placed in the evaporating dish and heated in a convection oven at about 120°C for about 10 minutes. The remaining VWR Brand MicroSlides were covered and allowed to cure overnight.

The next day, an about 3.5% solids solution of a polyamido-polyamine epichlorohydrin resin (described in US Patent No. 4,711,793) was made by adding the following to a 500mL flask and mixing thoroughly after each step in which a new ingredient was added:
about 4.4 g NaOH; then
about 407.5 g DI water; then
about 87.5 g 20% solids polyamido-polyamine epichlorohydrin resin (specifically 'Resicart E', made by Ciba-Geigy); and then
about 0.125 g TEPA (tetraethylenepentamine).

The VWR Brand MicroSlides were then submerged in the resin solution for about an hour. Solution was gently agitated on a rocker (Reliable Scientific Cat. #55) during submersion for better treatment uniformity. Upon removal of the VWR Brand MicroSlides from the solution, the VWR Brand MicroSlides were rinsed well with DI water and immediately placed on a metal hemi-drum.

Next, wet-as-cast porous nylon membrane (as described in U.S. Patent Nos. 3,876,738 and 4,707,265) was operatively positioned over the VWR Brand MicroSlides and stretched. The wet-as-cast porous nylon membrane was handled by personnel wearing gloves. The wet-as-cast porous nylon membrane used had been cast, quenched, and washed with DI water, but had not yet been exposed to a drying step, hence the term "wet-as-cast." The wet-as-cast porous nylon membrane had a nominal pore size of about 0.2 microns and a target initial bubble point of about 45 PSI (once dried). The base polymer for this wet-as-cast porous nylon membrane is Vydyne 66B nylon, which is a high molecular weight, standard amine nylon made by Solutia, Inc.

DI water was used to rinse the slides to remove any particles from the surface of the wet-as-cast porous nylon membrane/VWR Brand MicroSlide combination. During this process, it was found that leaving a layer of DI water on the VWR Brand MicroSlides before covering with the wet-as-cast porous nylon membrane enhanced the ability to apply and move the membrane around on the VWR Brand MicroSlides and, thus, to remove the air bubbles therebetween. During the application of the wet-as-cast porous nylon membrane to the treated VWR Brand MicroSlides slide, care was taken to ensure removal of any air bubbles between the wet-as-cast porous nylon membrane and each VWR Brand MicroSlide. The wet-as-cast porous nylon membrane was flattened onto each VWR Brand MicroSlide and all wrinkles were removed.

Once positioned on the VWR Brand MicroSlides, the wet-as-cast porous nylon membrane was clipped into position, as is known in the art. The entire assembly was then heated in a convection oven at about 120°C for about one hour. After heating, the excess now dried porous nylon membrane was removed from the VWR Brand MicroSlides by trimming, as is known in the art.

If the resultant nylon/glass composite slides were to be charge modified, they were then placed in an evaporating dish, and another, freshly made solution of about 3.5% solids polyamido-polyamine epichlorohydrin resin solution, was poured into the evaporating dish with the resultant nylon/glass composite slides. The resultant nylon/glass composite slides were allowed to remain submerged in the evaporating dish for about 5 minutes, then removed from the evaporating dish and rinsed with DI water. Most of the excess water was shaken off the resultant nylon/glass composite slides, and the resultant nylon/glass composite slides were placed into a dry evaporating dish and heated until dry in a convection oven at about 60°C for about twenty to thirty minutes.

The resulting nylon/glass composite slides (charged and uncharged) exhibited a very thin, smooth, layer of porous nylon membrane securely bound to the glass surface. The membrane surface appeared free of deformities, marks or particles.

When the resulting nylon/glass composite slides were tested in DI water, about 0.4M aqueous sodium hydroxide, or about a 1% aqueous sodium dodecyl sulfate (SDS), the nylon portion wetted readily. In general, the bond between the nylon portion and the glass portion remained strong, and the nylon could not be peeled away.

The nylon/glass bond of the resulting nylon/glass composite slides stayed strong even when the nylon/glass composite slides were quickly submerged vertically into boiling solutions of either DI water or SDS. Quick immersion into such boiling solutions does not allow the membrane to wet slowly, and high-pressure air bubbles can develop between the nylon membrane and the glass. Despite the harshness of the treatment, the uncharged glass/nylon composite slides retained their peel strength i.e., the membrane would rip before peeling from the glass. It was observed that the charged glass/nylon composites did not perform as well in SDS heated above about 65°C as the uncharged glass/nylon. In the charged glass/nylon composite slides, the nylon membrane sometimes delaminated from the glass as the bond between the nylon membrane and the glass surface appeared to exhibit weak bond strength. It is expected that further development in charge application methods, such as previously suggested, will provide even stronger bonds.

When the glass/nylon composites produced above were spotted with metanil yellow dye, the nylon membrane exhibited a uniform coloring throughout. The interface between the nylon membrane and the glass, because this interface contained the epichlorohydrin resin, became especially strongly colored. Specifically, it is believed that the positive charge of the quaternary amine groups in the polyamido-polyamine epichlorohydrin resin attracted the negatively charged dye molecules even more strongly than the amine groups in the nylon.

As can be seen from the above, this example demonstrates that a multi-cell substrate useful for carrying a microarray of biological polymers on the surface thereof has been produced using a wet-as-cast nylon membrane and a glass substrate by treating the glass substrate with a surface treatment that facilitates the covalent bonding of the wet-as-cast nylon membrane to the glass substrate in such a manner as to be useful in microarray applications.

### Example 2

Nylon/Glass Composite slides useful as a multi-cell substrate for carrying a microarray of biological polymers were produced as follows: In a second example, the glass slides are prepared and pre-treated in exactly the same way as in Example 1 in all respects except one. The one difference is that when the polyamido-polyamine epichlorohydrin solution was made up, no tetraethylene pentamine (TEPA) was added.

Upon testing the nylon/glass composite slides, it was found that the elimination of tetraethylene pentamine (TEPA) from the formulation produced a good bond between the glass and the nylon, and no tendency of the nylon portion to delaminate from the glass portion was observed when the composite was immersed in water at about 100°C.

### Example 3

This example describes the process for producing a sample of a nylon/Mylar composite useful as a multi-cell substrate for carrying a microarray of biological polymers. The nylon/Mylar composite is composed of a thin (~4 mil) porous nylon membrane bound to the surface of a sheet of Mylar.

During the production of the nylon/Mylar composites, the following solutions were prepared:
Solution 1: About a 100mL solution of about 2.5M H₂SO₄ was prepared. About 24.5g of fuming sulfuric acid was diluted with sufficient DI water to produce the about 100mL solution.
Solution 2: About a 3% glycidoxypropyltrimethoxysilane solution was prepared. About 3g of the chemical was diluted with sufficient DI water to produce the about 100mL solution.
Solution 3: About a 3.5% solids solution of a polyamido-polyamine epichlorohydrin resin (described in US Patent No. 4,711,793) was prepared by adding the following constituents to a 250mL flask and mixing thoroughly after each step:
   adding about 2.2g NaOH; then
   adding about 203.75g DI water; then
   adding about 43.75g 20% solids polyamido-polyamine epichlorohydrin resin (specifically 'Resicart E', made by Ciba-Geigy); and then
   adding about 0.125g TEPA (tetraethylenepentamine).

Next, about an eight-inch (8") x eleven-inch (11") Mylar sheet material was submerged in Solution 1 for about ten minutes. The Mylar sheet was removed from Solution 1, rinsed with DI water, and placed into Solution 2 for about ten minutes. The Mylar sheet was removed from Solution 2, rinsed with DI water and placed in Solution 3 for about 30 minutes.

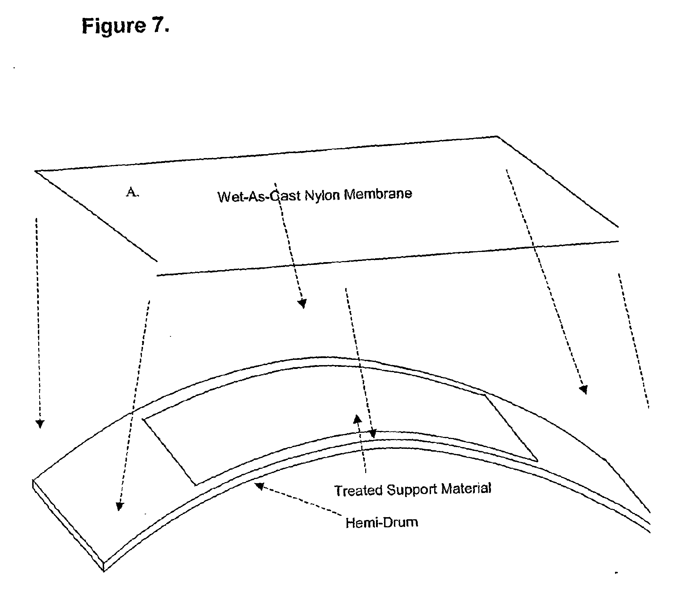

Upon removal from Solution 3, the Mylar sheet was rinsed well with DI water and immediately placed on a metal hemi-drum, similar to that illustrated in Figure 7.

Wet-as-cast porous nylon membrane (as described in U.S. Patents 3,876,738 and 4,707,265) was operatively positioned over the Mylar sheet and then the wet-as-cast porous nylon membrane was stretched. The wet-as-cast porous nylon membrane was handled by personnel wearing gloves. The nylon membrane used herein had been cast, quenched, and washed with DI water, but had not as yet been exposed to a drying step, hence the term "wet-as-cast." The wet-as-cast porous nylon membrane pore size was nominally 0.2 microns, with a target initial bubble point of about 45 PSI. The type of nylon used to produce the wet-as-cast porous nylon membrane was Vydyne 66B nylon, which is a high molecular weight, standard amine nylon made by Solutia, Inc. and is commercially available.

During the application of the wet-as-cast porous nylon membrane to the Mylar sheet, care was taken to ensure that any air bubbles between the nylon membrane and the Mylar were removed. The wet-as-cast porous nylon membrane was flattened and all wrinkles were removed. Before applying the wet-as-cast porous nylon membrane to the Mylar sheet, DI water was used to rinse the Mylar in order to remove any particles. During the process, it had been determined that leaving a layer of water on the Mylar sheet before covering it with the wet-as-cast porous nylon membrane enhanced the ability to move the membrane around and, thus, to remove the air bubbles therebetween.

Once applied to the Mylar sheet, the wet-as-cast porous nylon membrane was clipped into place on the hemi-drum, as is known in the art. The entire assembly was then heated in a convection oven at about 120°C for about one hour. After heating, the excess now dried porous nylon membrane was removed from the Mylar by trimming the edges of the dried porous nylon membrane away from the Mylar sheet, as is known in the art.

The resulting nylon/Mylar composite slide had a very thin, smooth, layer of porous nylon membrane securely bound to the surface of the Mylar sheet. The membrane surface appeared free of deformities, marks or particles.

When tested in about a 0.4M sodium hydroxide, and about 1% sodium dodecyl sulfate (SDS) in DI water, the porous nylon membrane wetted readily. After exposure to this solution, the bond between the nylon membrane portion and the Mylar portion remained strong, and the nylon membrane usually could not be peeled away without ripping the nylon.

The bond between the nylon membrane portion and the Mylar portion stayed strong even when the nylon/Mylar composite was quickly submerged vertically into boiling solutions of either water or SDS. As stated in an earlier example, quick immersion into such boiling solutions does not allow the nylon membrane to wet slowly, and high-pressure air bubbles can develop between the nylon membrane and the Mylar. Despite the harshness of the treatment, the nylon/Mylar composite retained its peel strength i.e., the nylon membrane would rip before peeling from the Mylar sheet.

In summary, several variations of the preferred method have resulted in permanent and robust bonding of nylon to various solid substrates, capable of withstanding the rigors of microarray applications, having the advantages of a thin and uniform functional layer of nylon useful for binding biomolecular analytes of DNA and other genomic products, proteins, etc., and suffering none of the drawbacks of the currently available microarray glass slides. Such drawbacks include having no membrane at all (functionalized glass), or the use of nitrocellulose as a less preferred membrane, or the use of a reinforced nylon having variable thickness characteristics, or the use of an adhesive layer between the membrane and the glass substrate.

### Prophetic Examples

Multi-cell substrate made by the phase inversion process and especially nylon membrane bound to a polymer substrate instead of glass has many potential microarray applications. The following is an attempt to describe processes for the production of such multi-cell substrates having a porous membrane formed by a phase inversion process operatively attached by covalent bonding through a surface treatment to a polymer substrate that prepares the substrate to be operatively, covalently bonded to the porous membrane formed by a phase inversion process such that the combination produced thereby is useful in microarray applications.

The following prophetic examples describe the steps that are believed necessary to produce nylon/non-porous support material composites other than the nylon/glass and nylon/Mylar composites that have been made (as described in Examples 1-3 above). The nylon/non-porous support material composites made would contain a thin (4 mil or less) porous nylon membrane bound to the surface of a non-porous support material.

As might be anticipated, different non-porous support materials must be pre-treated in different ways. The following list describes the pretreatments for different non-porous support materials:
1) Ceramic non-porous support material: Mix about 95 mL of ethanol, about 5 mL of water, and about 2 mL of 3-aminopropyl trimethoxysilane and let stand for about five minutes. Submerge the substrate into the solution for about two minutes, remove and rinse with ethanol. Heat the substrate for about 10 minutes at about 120°C, and let sit overnight. This particular solution should produce a great number of bonding sites for the nylon to the ceramic non-porous support material.
2) Acrylic non-porous support material: Acrylic polymers (acrylonitriles) contain nitrile bonds at most repeat units (not every repeat unit, as they tend to copolymerize). To prepare such support material for bonding with nylon, hydrolyze the nitriles to carboxylic acid groups by submerging the substrate in about 5M HCl (acid or base catalyzes the reaction) for about 10 minutes. This particular solution will produce a great number of bonding sites for the nylon to the acrylic polymers.
3) Polypropylene non-porous support material: Polypropylene is a relatively unreactive material. To make polypropylene open for bonding, treat the surface of the polypropylene with about a 0.4KW corona discharge. It is believed that the corona discharge may open up some bonding sites by producing carboxylic acid groups and carbonyl groups on the surface of the polypropylene non-porous support material. Because the effects of corona treatment may wear off over time, it is believed best to proceed to the next step, as described below, immediately. Alternatively, plasma treatment could also be used to introduce carboxyl or carbonyl groups into the surface which are suitable for bonding. Suitable gases for treatment may include helium, oxygen, acetylene, and carbon dioxide.
4) Polycarbonate and Polysulfone non-porous support material: The Polycarbonate and Polysulfone non-porous support material is placed in aqueous solution of about 1M NaOH with a bromine substituted carboxylic acid such as, for example, bromoacetic acid. The bromoacetic acid condenses with the phenol end groups of the polymer, releasing HBr as a side product. The resultant product of the condensation reaction has chains that now end with a carboxylic acid group that can then bond with the nylon.
5) Polyamide and Polyaramid non-porous support material: These polymers already contain carboxylic acid and amine end groups that can be used to react in the next step. They are presently believed not to require a pre-treatment.

Following the appropriate pre-treatment as described above, the respective non-porous support material is submerged in about a 3.5% solids solution of polyamido polyamine epichlorohydrin resin (hereafter referred to as the epichlorohydrin polymer). After about an hour, the respective non-porous support material is removed from the solution, and rinsed with DI water. The epichlorohydrin polymer should bond with the amino or carboxylic acid groups on the respective non-porous support material and the amino and carboxylic acid groups on the nylon, thereby bonding the nylon and the respective non-porous support material together.

After the respective non-porous support material is pre-treated as described above, wet-as-cast porous nylon membrane (as described in U.S. Patent Nos. 3,876,738 and 4,707,265) is placed over the respective non-porous support material and the wet-as-cast porous nylon membrane is stretched. The wet-as-cast porous nylon membrane is only handled by personnel wearing gloves. The wet-as-cast porous nylon membrane is obtained for applying to the respective non-porous support material after the wet-as-cast porous nylon membrane is cast, quenched, and washed with DI water, but has not yet been exposed to a drying step, hence the term "wet-as-cast." The type of polymer used is a high molecular weight, standard amine nylon.

Care is taken to remove any air bubbles between the wet-as-cast porous nylon membrane and the respective non-porous support material. The wet-as-cast porous nylon membrane is flattened on the respective non-porous support material and all wrinkles are removed from the wet-as-cast porous nylon membrane/respective non-porous support material combination. It is believed that rinsing the surface of the respective non-porous support material with DI water prior to applying the wet-as-cast porous nylon membrane will remove any particles on the respective non-porous support material. This step is thought to leave a layer of water on the surface of the respective non-porous support material before the respective non-porous support material is covered with the wet-as-cast porous nylon membrane and should facilitate movement of the wet-as-cast porous nylon membrane around relative to the surface of the respective non-porous support material thereby effectively removing any air bubbles that might form therebetween.

The wet-as-cast porous nylon membrane is then clipped into place on a hemi-drum. The entire assembly is heated in a convection oven at about 120°C for about one hour. After heating, the excess porous nylon membrane is removed from the respective non-porous support material by cutting away the edges of the porous nylon membrane from the respective non-porous support material, as is known in the art.

The resulting porous nylon membrane/respective non-porous support material composites should have a very thin, smooth layer of porous nylon membrane operatively bound to the respective non-porous support material. The porous nylon membrane surface should be free of deformities, marks or particles.

When tested in DI water, about 0.4M sodium hydroxide, and about 1% sodium dodecyl sulfate (SDS) in water, the nylon should wet readily. The bond between the porous nylon membrane component and the respective non-porous support material component of the resulting porous nylon membrane/respective non-porous support material composites should exhibit strong bonding, and the porous nylon membrane component should not peel away from the respective non-porous support material component.

The bond between the porous nylon membrane component and the respective non-porous support material component should stay strong even when the resulting porous nylon membrane/respective non-porous support material composites are quickly submerged vertically into boiling solutions of water or SDS. Despite the harshness of this treatment, the uncharged resulting porous nylon membrane/respective non-porous support material composites should retain their peel strength i.e., the porous nylon membrane component should rip before peeling away from the respective non-porous support material component.

The above prophetic examples are based on accepted principles of the chemistry of the various substrates (inorganic or organic polymers) and their surface chemistries, regarding the preparation of the surfaces for receptivity to the bifunctional linking chemistry as disclosed in the present application. These accepted principles of chemistry are not meant to be limitations on the preparations of the respective non-porous support material component. The accepted principles of chemistry are merely suggestions for defining starting points in the practice of the present disclosure, and may be modified by one skilled in the art, but still be in accordance with the inventive teachings of the present application.

As should be clear from the above examples and other description, the following chemicals have been found effective as silane surface treatments: 3-aminopropyl triethoxysilane, N-(2-aminoethyl)-3-aminopropyl trimethoxysilane, 3-glycidoxypropyltrimethoxysilane, and 2-(3,4-epoxycyclohexyl)-ethyltrimethoxysilane.

The following chemicals have been found effective as the second surface treatment: in general, a polyamido polyamine epichlorohydrin polymer, with the Resicart E polymer, made by Ciba Geigy, being a specific representative example of a polyamido polyamine epichlorohydrin polymer.

It should be apparent to those skilled in the art that there are significant portions of the glass or other non-porous support material and the nylon or other porous membrane formed by a phase inversion process which are in direct contact as opposed to having a third layer, such as, for example, glue or other definitive, separate adhesive substance separating the glass component and the nylon component so as to avoid a complete separate layer of material between the two as in at least some of the prior art. The specific pre-treatment material used to bond the glass or other non-porous support material component and the nylon or other porous membrane formed by a phase inversion process component has a negligible thickness, if measurable.

One clear distinction between the specific pre-treatment material used to bond the glass or other non-porous support material component and the nylon or other porous membrane formed by a phase inversion process component of the present application is that the specific pre-treatment material is distinguished from an adhesive as adhesives have particular properties which would in fact make a relatively determinable, thick layer between the two components.

It is also clear that when the glass or other non-porous support material component and the nylon or other porous membrane formed by a phase inversion process component of the present application is viewed, no third substance will be ascertainable as opposed to the prior art using an adhesive. The prior art using an adhesive clearly indicates a third substance. Thus, one prior art composite includes nylon, glass and a third substance holding the nylon and the glass together.

However, it is believed that an electron microscopic cross sectional analysis of the glass or other non-porous support material component and the nylon or other porous membrane formed by a phase inversion process component of the present composites will reveal no third substance between the two layers. Whereas it is believed that a distinct zone representing the adhesive of the prior art will be ascertained between the nylon and the glass of the prior art slide.

As should be apparent to those skilled in the art, nylon is the preferred substrate of use in nucleic acid detection assays. The reason that nylon is preferred over nitrocellulose is that nylon has a higher intrinsic positive charge. It is generally recognized that nylon, with its peptide backbone linkage, and well defined end-group chemistry, provides charge interactions which nitrocellulose cannot provide. Binding to nitrocellulose is dependent primarily on hydrophobic interactions. Binding in nylon is believed to be a function of charge. Additionally, nylon can be charged modified, thereby increasing the binding capacity of the nylon for nucleic acid, is much more robust than nitrocellulose, does not easily break, can be stripped and reprobed, is not an extreme fire hazard like nitrocellulose, is amenable to much more stringent washing and hybridization conditions.

The surface treatment of the present application has no discernable finite thickness or mass that could add nonuniformity to the overall thickness of the substrate/membrane combination structure and does not participate in the binding or detection of nucleic acid or protein analytes. This eliminates possible physical interference from the presence of an adhesive layer by precluding nonuniformity in thickness, and eliminates possible chemical interference by the absence of an additional substance that could participate in chemical reactions.

While the articles, apparatus and methods for making the articles contained herein constitute preferred embodiments of the invention, it is to be understood that the invention is not limited to these precise articles, apparatus and methods, and that changes may be made therein without departing from the scope of the invention which is defined in the appended claims.

## Claims

1. A method of fabricating a multi-cell substrate useful for carrying a microarray of biological polymers comprising the acts of:
providing a non-porous substrate;
providing a microporous membrane formed by a phase inversion process;
providing a surface treatment;
applying the surface treatment to the non-porous substrate; and
intermingling the non-porous substrate having the surface treatment with the microporous membrane such that the non-porous substrate is sufficiently covalently bonded to the microporous membrane wherein the combination produced thereby is useful in microarray applications.

2. The method of claim 1 wherein the surface treatment is selected from the group comprising:
3-aminopropyl triethoxysilane, N-(2-aminoethyl)-3-aminopropyl trimethoxysilane, 3-glycidoxypropyltrimethoxysilane, (10-carbomethoxydecyl) dimethylchlorosilane or 2-(3,4-epoxycyclohexyl)-ethyltrimethoxysilane.

3. The method of claim 1 wherein, the surface treatment comprises a 3-aminopropyl triethoxysilane followed by treatment with a polyamido-polyamine epichlorohydrin resin.

4. The method of claim 1 wherein, the non-porous substrate is selected from the group comprising:
glass, Mylar, ceramic, acrylic, polypropylene, polycarbonate, polysulfone, polyamide and polyaramid.

5. The method of claim 1 wherein, the non-porous substrate is glass.

6. The method of claim 1 wherein, the non-porous substrate is a polyester.

7. The method of claim 1 wherein, the non-porous substrate is Mylar.

8. The method of claim 7 wherein, the surface of the Mylar is oxidized with sulfuric acid or corona discharge to enable it to bond to a polyamido-polyamine epichlorohydrin polymer.

9. A multi-cell substrate, useful for carrying a microarray of biological polymers comprising:
a microporous membrane formed by a phase inversion process;
a non-porous substrate; and
a surface treatment, operatively positioned between the microporous membrane and the non-porous substrate, for sufficiently covalently bonding the non-porous substrate to the microporous membrane wherein the combination multi-cell substrate produced thereby is useful in microarray applications.

10. The multi-cell substrate of claim 9 wherein, the surface treatment is selected from the group comprising:
3-aminopropyl triethoxysilane, N-(2-aminoethyl)-3-aminopropyltrimethoxysilane, 3-glycidoxypropyltrimethoxysilane, (10-carbomethoxydecyl) dimethylchlorosilane or 2-(3,4-epoxycyclohexyl)-ethyltrimethoxysilane.

11. The multi-cell substrate of claim 9 wherein, the non-porous substrate is selected from the group comprising:
glass, Mylar, ceramic, acrylic, polypropylene, polycarbonate, polysulfone, polyamide and polyaramid.

12. The multi-cell substrate of claim 9 wherein, the surface treatment comprises:
a 3-aminopropyl triethoxysilane followed by treatment with a polyamido-polyamine epichlorohydrin resin.

13. The multi-cell substrate of claim 9 wherein, the non-porous substrate is glass.

14. The multi-cell substrate of claim 9 wherein, the non-porous substrate is a polyester.

15. The multi-cell substrate of claim 9 wherein the, the non-porous substrate is Mylar.

16. The multi-cell substrate of claim 9 wherein the phase inversion membrane is selected from the group consisting of:
nylon 66, nylon 46, nylon 6, polysulfone, polyethersulfone and polyvinylidenediflouride (PVDF).

17. The method of claim 1 wherein the phase inversion membrane is selected from the group consisting of:
nylon 66, nylon 46, nylon 6, polysulfone, polyethersulfone and polyvinylidenediflouride (PVDF).

18. The multi-cell substrate of claim 9 wherein the surface treatment has no discernable finite thickness or mass which could add nonuniformity to the overall thickness of the multi-cell substrate.

19. The multi-cell substrate of claim 9 wherein the surface treatment minimizes participation in the binding or detection of nucleic acid or protein analytes.

20. The multi-cell substrate of claim 9 wherein the surface treatment minimizes the interference of the substances used to connect the solid substrate portion to the porous membrane portion thereof with the detection of analytes.

21. The multi-cell substrate of claim 9 wherein the surface treatment at least substantially eliminates nonuniformity of the overall thickness of the substrate/membrane combination structure.

## Patentansprüche

1. Verfahren zur Herstellung eines mehrzelligen Substrats, das brauchbar ist, um ein Mikroarray biologischer Polymere zu tragen, umfassend die Schritte:
Bereitstellen eines nicht porösen Substrats;
Bereitstellen einer nach einem Phasenumkehrverfahren gebildeten mikroporösen Membran;
Bereitstellen einer Oberflächenbehandlung;
Anwenden der Oberflächenbehandlung auf das nicht poröse Substrat, und
Vermischen des nicht porösen Substrats, das die Oberflächenbehandlung erhalten hat, mit der mikroporösen Membran, so dass das nicht poröse Substrat in ausreichendem Maße kovalent an die mikroporöse Membran gebunden wird, wobei die hierdurch produzierte Kombination in Mikroarrayanwendungen brauchbar ist.

2. Verfahren nach Anspruch 1, wobei die Oberflächenbehandlung aus der Gruppe umfassend 3-Aminopropyltriethoxysilan, N-(2-Aminoethyl)-3-aminopropyltrimethoxysilan, 3-Glycidoxypropyltrimethoxysilan, (10-Carbomethoxydecyl)dimethylchlorsilan oder 2-(3,4-Epoxycyclohexyl)ethyltrimethoxysilan ausgewählt ist.

3. Verfahren nach Anspruch 1, wobei die Oberflächenbehandlung ein 3-Aminopropyltriethoxysilan umfasst, gefolgt von einer Behandlung mit einem Polyamido-Polyamin-Epichlorhydrinharz.

4. Verfahren nach Anspruch 1, wobei das nicht poröse Substrat aus der Gruppe umfassend Glas, Mylar, Keramik, Acrylharz, Polypropylen, Polycarbonat, Polysulfon, Polyamid und Polyaramid ausgewählt ist.

5. Verfahren nach Anspruch 1, wobei das nicht poröse Substrat Glas ist.

6. Verfahren nach Anspruch 1, wobei das nicht poröse Substrat ein Polyester ist.

7. Verfahren nach Anspruch 1, wobei das nicht poröse Substrat Mylar ist.

8. Verfahren nach Anspruch 7, wobei die Oberfläche des Mylars mit Schwefelsäure oder Koronaentladung oxidiert wird, damit sie an ein Polyamido-Polyamin-Epichlorhydrinpolymer binden kann.

9. Mehrzelliges Substrat, das brauchbar ist, um ein Mikroarray von biologischen Polymeren zu tragen, umfassend:
eine nach einem Phasenumkehrverfahren gebildete mikroporöse Membran;
ein nicht poröses Substrat und
eine Oberflächenbehandlung, die in Wirkbeziehung zwischen der mikroporösen Membran und dem nicht porösen Substrat angeordnet ist, um das nicht poröse Substrat in ausreichendem Maße kovalent an die mikroporöse Membran zu binden, wobei das **dadurch** produzierte mehrzellige Kombinationssubstrat in Mikroarrayanwendungen brauchbar ist.

10. Mehrzelliges Substrat nach Anspruch 9, wobei die Oberflächenbehandlung aus der Gruppe umfassend 3-Aminopropyltriethoxysilan, N-(2-Aminoethyl)-3-aminopropyltrimethoxysilan, 3-Glycidoxypropyltrimethoxysilan, (10-Carbomethoxydecyl)dimethylchlorsilan oder 2-(3,4-Epoxycyclohexyl)ethyltrimethoxysilan ausgewählt ist.

11. Mehrzelliges Substrat nach Anspruch 9, wobei das nicht poröse Substrat aus der Gruppe umfassend Glas, Mylar, Keramik, Acrylharz, Polypropylen, Polycarbonat, Polysulfon, Polyamid und Polyaramid ausgewählt ist.

12. Mehrzelliges Substrat nach Anspruch 9, wobei die Oberflächenbehandlung ein 3-Aminopropyltriethoxysilan umfasst, gefolgt von einer Behandlung mit einem Polyamido-Polyamin-Epichlorhydrinharz.

13. Mehrzelliges Substrat nach Anspruch 9, wobei das nicht poröse Substrat Glas ist.

14. Mehrzelliges Substrat nach Anspruch 9, wobei das nicht poröse Substrat ein Polyester ist.

15. Mehrzelliges Substrat nach Anspruch 9, wobei das nicht poröse Substrat Mylar ist.

16. Mehrzelliges Substrat nach Anspruch 9, wobei die Phasenumkehrmembran aus der Gruppe bestehend aus Nylon 66, Nylon 46, Nylon 6, Polysulfon, Polyethersulfon und Polyvinylidendifluorid (PVDF) ausgewählt ist.

17. Verfahren nach Anspruch 1, wobei die Phasenumkehrmembran aus der Gruppe bestehend aus Nylon 66, Nylon 46, Nylon 6, Polysulfon, Polyethersulfon und Polyvinylidendifluorid (PVDF) ausgewählt ist.

18. Mehrzelliges Substrat nach Anspruch 9, wobei die Oberflächenbehandlung keine wahrnehmbare endliche Dicke oder Masse hat, die der Gesamtdicke des mehrzelligen Substrats Uneinheitlichkeit verleihen könnte.

19. Mehrzelliges Substrat nach Anspruch 9, wobei die Oberflächenbehandlung das Partizipieren an der Bindung oder Detektion von Nukleinsäure- oder Proteinanalyten minimiert.

20. Mehrzelliges Substrat nach Anspruch 9, wobei die Oberflächenbehandlung die Interferenz von Substanzen, die zum Verbinden des festen Substratanteils mit dem porösen Membrananteil verwendet worden sind, mit der Detektion von Analyten minimiert.

21. Mehrzelliges Substrat nach Anspruch 9, wobei die Oberflächenbehandlung Ungleichförmigkeit der Gesamtdicke der Substrat/Membran-Kombinationsstruktur mindestens im Wesentlichen eliminiert.

## Revendications

1. Procédé de fabrication d'un substrat multicellulaire utile pour porter une micromatrice de polymères biologiques comprenant les étapes consistant à :
fournir un substrat non poreux ;
fournir une membrane microporeuse formée par un procédé d'inversion de phase ;
fournir un traitement de surface ;
appliquer le traitement de surface au substrat non poreux ; et
entremêler le substrat non poreux ayant subi le traitement de surface avec la membrane microporeuse de sorte que le substrat non poreux est suffisamment lié de manière covalente à la membrane microporeuse, dans lequel la combinaison ainsi produite est utile dans des applications de micromatrices.

2. Procédé selon la revendication 1, dans lequel le traitement de surface est choisi parmi le groupe constitué :
de 3-aminopropyltriéthoxysilane, de N-(2-aminoéthyl)-3-aminopropyltriméthoxysilane, 3-glycidoxypropyltriméthoxysilane, (10-carbométhoxydécyl) diméthylchlorosilane ou 2-(3,4-époxycyclohexyl)-éthyltriméthoxysilane.

3. Procédé selon la revendication 1, dans lequel le traitement de surface comprend un 3-aminopropyltriéthoxysilane suivi d'un traitement avec une résine de polyamido-polyamine épichlorohydrine.

4. Procédé selon la revendication 1, dans lequel le substrat non poreux est choisi parmi le groupe constitué :
de verre, de mylar, de céramique, d'acrylique, de polypropylène, de polycarbonate, de polysulfone, de polyamide et de polyaramide.

5. Procédé selon la revendication 1, dans lequel le substrat non poreux est du verre.

6. Procédé selon la revendication 1, dans lequel le substrat non poreux est un polyester.

7. Procédé selon la revendication 1, dans lequel le substrat non poreux est du mylar.

8. Procédé selon la revendication 7, dans lequel la surface du mylar est oxydée avec de l'acide sulfurique ou une décharge corona pour lui permettre de se lier à un polymère de polyamido-polyamine épichlorohydrine.

9. Substrat multicellulaire utile pour porter une micromatrice de polymères biologiques comprenant :
une membrane microporeuse formée par un procédé d'inversion de phase ;
un substrat non poreux ; et
un traitement de surface, positionné de manière fonctionnelle entre la membrane microporeuse et le substrat non poreux, pour lier suffisamment de manière covalente le substrat non poreux à la membrane microporeuse, dans lequel la combinaison de substrat multicellulaire ainsi produite est utile dans des applications de micromatrices.

10. Substrat multicellulaire selon la revendication 9, dans lequel le traitement de surface est choisi parmi le groupe constitué :
de 3-aminopropyltriéthoxysilane, de N-(2-aminoéthyl)-3-aminopropyltriméthoxysilane, 3-glycidoxypropyltriméthoxysilane, (10-carbométhoxydécyl) diméthylchlorosilane ou 2-(3,4-époxycyclohexyl)-éthyltriméthoxysilane.

11. Substrat multicellulaire selon la revendication 9, dans lequel le substrat non poreux est choisi parmi le groupe constitué :
de verre, de mylar, de céramique, d'acrylique, de polypropylène, de polycarbonate, de polysulfone, de polyamide et de polyaramide.

12. Substrat multicellulaire selon la revendication 9, dans lequel le traitement de surface comprend :
un 3-aminopropyltriéthoxysilane suivi d'un traitement avec une résine de polyamido-polyamine épichlorohydrine.

13. Substrat multicellulaire selon la revendication 9, dans lequel le substrat non poreux est du verre.

14. Substrat multicellulaire selon la revendication 9, dans lequel le substrat non poreux est un polyester.

15. Substrat multicellulaire selon la revendication 9, dans lequel le substrat non poreux est du mylar.

16. Substrat multicellulaire selon la revendication 9, dans lequel la membrane d'inversion de phase est choisie parmi le groupe constitué :
du nylon 66, du nylon 46, du nylon 6, d'un polysulfone, d'un polyéthersulfone et d'un polyvinylidènedifluorure (PVDF).

17. Procédé selon la revendication 1, dans lequel la membrane d'inversion de phase est choisie parmi le groupe constitué :
du nylon 66, du nylon 46, du nylon 6, d'un polysulfone, d'un polyéthersulfone et d'un polyvinylidènedifluorure (PVDF).

18. Substrat multicellulaire selon la revendication 9, dans lequel le traitement de surface ne possède aucune épaisseur ou masse finie perceptible qui pourrait réduire l'uniformité de l'épaisseur totale du substrat multicellulaire.

19. Substrat multicellulaire selon la revendication 9, dans lequel le traitement de surface minimise la participation dans la liaison ou la détection d'analytes d'acides nucléiques ou de protéines.

20. Substrat multicellulaire selon la revendication 9, dans lequel le traitement de surface minimise la perturbation des substances utilisées pour relier la partie de substrat solide à la partie de membrane poreuse de celui-ci avec la détection d'analytes.

21. Substrat multicellulaire selon la revendication 9, dans lequel le traitement de surface élimine au moins en grande partie le manque d'uniformité de l'épaisseur totale de la structure de la combinaison substrat/membrane.
